# EUROPEAN PATENT SPECIFICATION

(11) **EP 4 467 966 B1**
(45) Date of publication and mention of the grant of the patent: **30.09.2026**
(21) Application number: 24178090.7
(22) Date of filing: 24.05.2024
(51) Int. Cl.: G01N 21/31, G01N 21/84, A01C 21/00, G01N 33/00, G06N 3/08, G06N 20/00, G01N 21/359

(54) **METHOD, COMPUTER READABLE MEDIUM AND SYSTEM FOR DETERMINING REFINED PETIOLE NUTRIENT VALUES BASED ON LEAF SPECTRAL DATA**
VERFAHREN, COMPUTERLESBARES MEDIUM UND SYSTEM ZUR BESTIMMUNG VERFEINERTER ERNÄHRUNGSWERTE AUF BASIS VON BLATTSPEKTRALDATEN
PROCÉDÉ, SUPPORT LISIBLE PAR ORDINATEUR ET SYSTÈME POUR LA DÉTERMINATION DE VALEURS NUTRITIVES DE PETILE RAFFINÉ SUR LA BASE DE DONNÉES SPECTRALES DE FEUILLES

(30) Priority: 25.05.2023 US 202318323490; 05.04.2024 US 202418627504
(43) Date of publication of application: 27.11.2024
(73) Proprietor: Dalhousie University, Halifax, Nova Scotia B3H 4R2 (CA)
(72) Inventor: Al-Mallahi, Ahmad Ali Falah, Truro, B2N 5E3 (CA); Abukmeil, Reem, Truro, B2N 5E3 (CA); Pinto, Felipe Campelo Franca, Birmingham, B4 7ET (GB)
(74) Representative: Greaves Brewster LLP

(56) References cited:
- CN-A- 114 509 403
- US-B1- 6 683 970
- ABUKMEIL REEM ET AL: "New approach to estimate macro and micronutrients in potato plants based on foliar spectral reflectance", vol. 198, 28 May 2022 (2022-05-28), AMSTERDAM, NL, pages 107074, XP093215525, ISSN: 0168-1699, Retrieved from the Internet <URL:https://www.sciencedirect.com/science/article/pii/S016816992200391X/pdfft?md5=109f6314aaba4383ebc3869fdd3b2c45&pid=1-s2.0-S016816992200391X-main.pdf> DOI: 10.1016/j.compag.2022.107074
- COHEN Y ET AL: "Leaf nitrogen estimation in potato based on spectral data and on simulated bands of the VENÎ1?4S satellite", PRECISION AGRICULTURE, KLUWER ACADEMIC PUBLISHERS, BO, vol. 11, no. 5, 12 November 2009 (2009-11-12), pages 520 - 537, XP019828812, ISSN: 1573-1618
- JONGSCHAAP RAYMOND E E ET AL: "Spectral measurements at different spatial scales in potato: relating leaf, plant and canopy nitrogen status", INTERNATIONAL JOURNAL OF APPLIED EARTH OBSERVATION AND GEOINFORMATION, ELSEVIER, AMSTERDAM, NL, vol. 5, no. 3, 6 August 2004 (2004-08-06), pages 205 - 218, XP087029361, ISSN: 1569-8432, [retrieved on 20040806], DOI: 10.1016/J.JAG.2004.03.002

## Description

### FIELD

This document relates to systems, apparatus, and methods of determining petiole nutrient values based on leaf spectral data from plant leaves.

### BACKGROUND

Plants need many nutrients for growth and sustenance. For example, plants may need macronutrients like nitrogen (N), phosphorus (P), potassium (K), calcium (Ca), magnesium (Mg), and sulfur (S) in large quantities. Plants may also need micronutrients like manganese (Mn), zinc (Zn), iron (Fe), sodium (Na), copper (Cu), aluminum (Al), and boron (B) in small quantities.

In agricultural applications, crop yield and/or quality of harvested crops may be affected if the crop plants do not receive a suitable amount of each required nutrient. For example, the crop yield and/or quality may be reduced if the plants do not receive enough nutrients. In some cases, excessive supply of nutrients may also reduce the crop yield and/or quality.

Determining the nutrient value status of plants can enable corrective actions to be performed to improve crop yield and/or quality. For example, the composition and quantity of fertilizer may be tailored for one or more specific nutrients based on the determined nutrient value status of the plants.

"New approach to estimate macro and micronutrients in potato plants based on foliar spectral reflectance" by Abukmeil Reem et al., COMPUTERS AND ELECTRONICS IN AGRICULTURE, vol. 198, 28 May 2022, page 107074, discloses an assessment of correlation between the nutrients of potato petioles and leaf spectrum.

### SUMMARY

The invention is defined by the method of claim 1, the non-transitory computer-readable medium of claim 5 and the system of claim 9.

The following summary is provided to introduce the reader to the more detailed discussion to follow.

According to some embodiments, a method of non-destructively determining petiole nutrient values in near real-time from plant leaves in a crop field using a portable spectrophotometer is provided. The method may comprise: (a) taking non-destructive spectral measurements of a leaf of a plant in the crop field using the portable spectrophotometer to generate leaf spectral data, (b) storing the leaf spectral data in a memory; (c) computing, by a processor, the petiole nutrient values based on the stored leaf spectral data; and (d) providing a near real-time result indicating the petiole nutrient values.

According to some embodiments, an apparatus for non-destructively determining petiole nutrient values in near real-time from plant leaves in a crop field is provided. The apparatus may comprise: a memory storing one or more machine learning models trained to provide petiole nutrient value outputs based on leaf spectral data inputs; and at least one processor communicatively coupled with the memory. The at least one processor may be configured to collectively: receive leaf spectral data generated by a portable spectrophotometer based on non-destructive spectral measurements of a leaf of a plant in the crop field; store the leaf spectral data in the memory; input the leaf spectral data into the one or more machine learning models; receive an output comprising the petiole nutrient values determined by the one or more machine learning models based on the leaf spectral data; and provide a near real-time result to a mobile device, the near real-time result indicating the petiole nutrient values.

According to some embodiments, a non-transitory computer-readable medium comprising instructions executable by a processor is provided. The instructions when executed may configure the processor to: receive leaf spectral data generated by a portable spectrophotometer based on non-destructive spectral measurements of a leaf of a plant in a crop field; store the leaf spectral data in a memory; input the leaf spectral data into one or more machine learning models trained to provide petiole nutrient value outputs based on leaf spectral data inputs; receive an output comprising the petiole nutrient values determined by the one or more machine learning models based on the leaf spectral data; and provide a near real-time result indicating the petiole nutrient values.

According to some embodiments, a system for non-destructively determining petiole nutrient values in near real-time from plant leaves in a crop field is provided. The system may comprise a portable spectrophotometer and a remote server. The portable spectrophotometer may be configured to: generate leaf spectral data based on non-destructive spectral measurements of a leaf of a plant in the crop field; and transmit the leaf spectral data for receipt by the remote server via a network. The remote server may comprise a processor and a memory, the memory storing one or more machine learning models trained to provide petiole nutrient value outputs based on leaf spectral data inputs. The remote server may be configured to: receive the leaf spectral data; store the leaf spectral data in the memory; input the leaf spectral data into the one or more machine learning model; receive the petiole nutrient values determined by the one or more machine learning model based on the leaf spectral data; and provide a near real-time result indicating the petiole nutrient values to a mobile device.

The invention concerns a method of determining refined petiole nutrient values based on leaf spectral data from plant leaves is provided. The method comprises:
(a) storing the leaf spectral data in a memory, (b) transforming, using a processor, the leaf spectral data into preliminary petiole nutrient values by inputting the leaf spectral data into one or more linear machine learning models trained to provide petiole nutrient value outputs based on leaf spectral data inputs, the one or more linear machine learning models outputting the preliminary petiole nutrient values; (c) refining, using the processor, the preliminary petiole nutrient values by inputting the preliminary petiole nutrient values into one or more non-linear machine learning models trained to provide refined petiole nutrient value outputs based on preliminary petiole nutrient value inputs, the one or more non-linear machine learning models outputting refined petiole nutrient values; and (d) outputting a plant nutrient report indicative of the refined petiole nutrient values.

The invention also concerns a non-transitory computer-readable medium comprising instructions executable by a processor is provided. The instructions when executed configure the processor to: store leaf spectral data from plant leaves in a memory; transform, using the processor, the leaf spectral data into preliminary petiole nutrient values by inputting the leaf spectral data into one or more linear machine learning models trained to provide petiole nutrient value outputs based on leaf spectral data inputs; receive an output comprising the preliminary petiole nutrient values from the one or more linear machine learning models; refine, using the processor, the preliminary petiole nutrient values by inputting the preliminary petiole nutrient values into one or more non-linear machine learning models trained to provide refined petiole nutrient value outputs based on preliminary petiole nutrient value inputs; receive an output comprising the refined petiole nutrient values from the one or more non-linear machine learning models; and output a plant nutrient report indicative of the refined petiole nutrient values.

The invention also concerns a system for determining refined petiole nutrient values based on leaf spectral data from plant leaves is provided. The system comprises a memory and a processor. The memory is configured to store leaf spectral data from plant leaves. The processor is configured to: transform the leaf spectral data into preliminary petiole nutrient values by inputting the leaf spectral data into one or more linear machine learning models trained to provide petiole nutrient value outputs based on leaf spectral data inputs; receive an output comprising the preliminary petiole nutrient values from the one or more linear machine learning models; refine the preliminary petiole nutrient values by inputting the preliminary petiole nutrient values into one or more non-linear machine learning models trained to provide refined petiole nutrient value outputs based on preliminary petiole nutrient value inputs; receive an output comprising the refined petiole nutrient values from the one or more non-linear machine learning models; and output a plant nutrient report indicative of the refined petiole nutrient values.

### BRIEF DESCRIPTION OF THE DRAWINGS

The drawings included herewith are for illustrating various examples of articles, methods, and apparatuses of the present specification and are not intended to limit the scope of what is taught in any way. In the drawings:
FIG. 1 is an illustration of a leaf of a plant whose nutrient values may be determined, in accordance with an embodiment;
FIG. 2 is a schematic illustration of a system for non-destructively determining petiole nutrient values in near real-time from plant leaves in a crop field, in accordance with an embodiment;
FIG. 3 is a schematic illustration of a result indicating petiole nutrient values provided by the system of FIG. 2 and graphically displayed on a mobile device;
FIG. 4 is a schematic illustration of an apparatus for non-destructively determining petiole nutrient values in near real-time from plant leaves in a crop field, in accordance with an embodiment;
FIG. 5 is a flowchart illustrating an example method of non-destructively determining petiole nutrient values in near real-time from plant leaves in a crop field using a portable spectrophotometer;
FIG. 6 is a table illustrating interrelations in nutrient uptake characteristics of potato plants in accordance with an embodiment; and
FIG. 7 is a flowchart illustrating an example method of determining refined petiole nutrient values based on leaf spectral data from plant leaves, in accordance with the invention.

### DETAILED DESCRIPTION

Numerous embodiments are described in this application and are presented for illustrative purposes only. The described embodiments are not intended to be limiting in any sense. The invention is widely applicable to numerous embodiments, as is readily apparent from the disclosure herein. Those skilled in the art will recognize that the present invention may be practiced with modification and alteration without departing from the teachings disclosed herein. Although particular features of the present invention may be described with reference to one or more particular embodiments or figures, it should be understood that such features are not limited to usage in the one or more particular embodiments or figures with reference to which they are described.

The terms "an embodiment," "embodiment," "embodiments," "the embodiment," "the embodiments," "one or more embodiments," "some embodiments," and "one embodiment" mean "one or more (but not all) embodiments of the present invention(s)," unless expressly specified otherwise.

The terms "including," "comprising" and variations thereof mean "including but not limited to," unless expressly specified otherwise. A listing of items does not imply that any or all of the items are mutually exclusive, unless expressly specified otherwise. The terms "a," "an" and "the" mean "one or more," unless expressly specified otherwise.

**As** used herein and in the claims, two or more parts are said to be "coupled", "connected", "attached", "joined", "affixed", or "fastened" where the parts are joined or operate together either directly or indirectly (i.e., through one or more intermediate parts), so long as a link occurs. As used herein and in the claims, two or more parts are said to be "directly coupled", "directly connected", "directly attached", "directly joined", "directly affixed", or "directly fastened" where the parts are connected in physical contact with each other. As used herein, two or more parts are said to be "rigidly coupled", "rigidly connected", "rigidly attached", "rigidly joined", "rigidly affixed", or "rigidly fastened" where the parts are coupled so as to move as one while maintaining a constant orientation relative to each other. None of the terms "coupled", "connected", "attached", "joined", "affixed", and "fastened" distinguish the manner in which two or more parts are joined together.

Further, although method steps may be described (in the disclosure and / or in the claims) in a sequential order, such methods may be configured to work in alternate orders. In other words, any sequence or order of steps that may be described does not necessarily indicate a requirement that the steps be performed in that order. The steps of methods described herein may be performed in any order that is practical. Further, some steps may be performed simultaneously.

As used herein and in the claims, a group of elements are said to 'collectively' perform an act where that act is performed by any one of the elements in the group, or performed cooperatively by two or more (or all) elements in the group.

As used herein and in the claims, a first element is said to be "received" in a second element where at least a portion of the first element is received in the second element unless specifically stated otherwise.

Some elements herein may be identified by a part number, which is composed of a base number followed by an alphabetical or subscript-numerical suffix (e.g., 112a, or 112₁). Multiple elements herein may be identified by part numbers that share a base number in common and that differ by their suffixes (e.g., 112₁, 112₂, and 112₃). All elements with a common base number may be referred to collectively or generically using the base number without a suffix (e.g., 112).

As used herein and in the claims, "up", "down", "above", "below", "upwardly", "vertical", "elevation", "upper", "lower" and similar terms are in reference to a directionality generally aligned with (e.g., parallel to) gravity. The terms "distal", "proximal" and similar terms are in reference to a directionality generally that is transverse (e.g., perpendicular) to gravity. However, none of the terms referred to in this paragraph imply any particular alignment between elements. For example, a first element may be said to be "vertically above" a second element, where the first element is at a higher elevation than the second element, and irrespective of whether the first element is vertically aligned with the second element.

Generally, plant tissue tests for determining plant nutrient value status are destructive and do not provide real-time results. For example, a farmer may harvest petioles from plants in a crop field for measurement. The harvested petioles may be shipped to a laboratory for testing. Depending on the testing to be performed, a large number of petioles may need to be destructively removed.

The laboratory may then conduct plant tissue testing and provide petiole nutrient values to the farmer. The process of removing the petioles, shipping, testing, and result notification may take weeks to complete. This delay undermines the effectiveness of any corrective action (e.g. adjusting fertilizer composition and quantity) taken based on the results because the nutrient content in the measured plants may have changed in the intervening period.

The disclosed systems, apparatus and methods can provide non-destructive and near real-time determination of plant nutrient value status using a portable spectrophotometer. Molecules absorb light at frequencies that are characteristic of their structure. The portable spectrophotometer can be used to take non-destructive spectral measurements that indicate the presence and concentration of different molecules (nutrients) in the probed region (e.g., a leaf of a plant). The portable spectrophotometer can generate leaf spectral data (based on the non-destructive spectral measurements) that can be used to determine the nutrient value status of plants. No plant leaves or petioles are required to be removed from the plants for testing. The disclosed system, apparatus and method can provide the plant nutrient value status without destruction of any of the plants used for testing.

Further, the disclosed system, apparatus and method can conduct the analysis and provide the results in near real-time, for example, within 10-20 seconds. This can enable any required corrective actions (based on the determined plant nutrient value status) to be immediately performed. As used herein and in the claims, an action is said to be performed in "near real-time" where that action is performed in less than 1 minute from when it was initiated.

In canopy reflectance measurements, ground-based or air-borne platforms are used to determine the ratio of the amount of light leaving the canopy to the amount of incoming light. The canopy reflectance measurement may be used for calculating the crop nutrient value status. However, canopy reflectance measurements are frequently subject to atmospheric and soil interference. The disclosed system, apparatus and method can use a portable spectrophotometer to probe plant leaves which avoids the atmospheric interference and/or soil interference associated with conducting canopy reflectance measurements using ground-based or air-borne platforms.

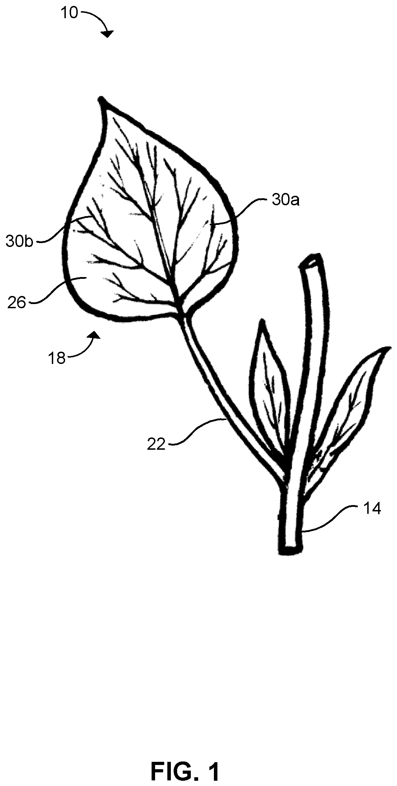

Referring now to FIG. 1, shown therein is an illustration of a plant 10 whose nutrient value status may be determined using the disclosed system, apparatus or method. In the illustrated example, plant 10 includes a stem 14, a leaf 18 and a petiole 22 attaching leaf 18 to stem 14. Leaf 18 includes a leaf blade 26 and multiple leaf veins 30.

The surface area of leaf blade 26 can be much larger compared with petiole 22 and therefore spectral reflectance measurements may be taken from leaf blade 26 instead of petiole 22. Accordingly, the measured nutrient values may be indicative of the nutrient values corresponding to the leaf blade instead of the petiole nutrient values.

Nutrient values in petioles are not the same as nutrient values measured at other parts of a plant (e.g. the leaf blade). At present and historically, farmers have sent petioles (specifically, the fourth petiole counting from the upper tip of the plant) to laboratories, and the laboratories have returned petiole nutrient values. As such, farmers generally understand what corrective actions are required based on laboratory petiole nutrient values but not based on nutrient values associated with other parts of the plant.

The disclosed system, apparatus and method can take spectral measurements from leaf blades of a plant to produce leaf spectral data and compute petiole nutrient values based on the leaf spectral data. Providing petiole nutrient values (as opposed to nutrient values associated with the leaf) can enable a user to select and apply well known and understood corrective actions.

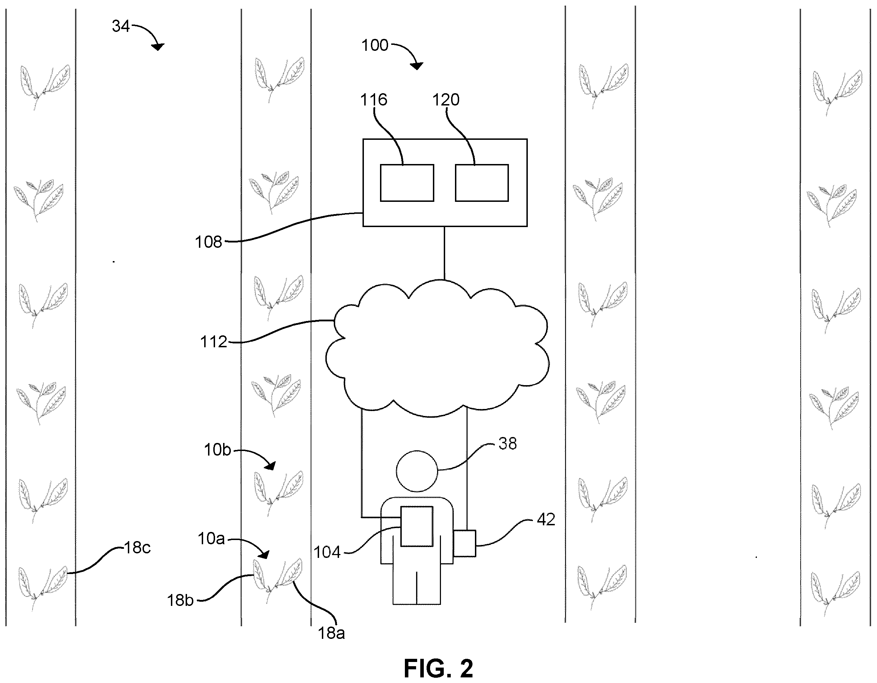

Referring now to FIG. 2, shown therein is a schematic illustration of a system 100 for non-destructively determining petiole nutrient values in near real-time from plant leaves 18 in a crop field 34, in accordance with an embodiment. As shown, system 100 may include a portable spectrophotometer 104 and a remote server 108.

In the illustrated example, portable spectrophotometer 104 and remote server 108 are communicatively coupled using a network 112. Network 112 may include a communication network such as the Internet, a Wide-Area Network (WAN), a Local-Area Network (LAN), or another type of network. In other examples, portable spectrophotometer 104 and remote server 108 may not be connected to a common network and may communicate using intermediary devices and/or communication networks.

Crop field 34 may include multiple plants 10 (e.g., at least 500 plants). Each plant 10 may have one or more leaves 18. Plants 10 may include any suitable plants grown in agricultural applications. For example, plants 10 may include wheat, corn, soy, potato etc. Crop field 34 may be located in open air or inside a greenhouse.

Portable spectrophotometer 104 may have any suitable design that is portable and capable of taking non-destructive spectral measurements of leaves of plants 10. Portable spectrophotometer 104 may be sized and designed to be carried by an operator while the operator uses the portable spectrophotometer to take spectral measurements. For example, portable spectrophotometer 104 may be battery-operated and designed to be carried in a backpack by an operator 38.

A light source within portable spectrophotometer 104 can generate incident light onto a leaf and a detector within portable spectrophotometer 104 can measure the light reflected from leaves of plants 10. Portable spectrophotometer 104 may generate leaf spectral data based on the reflected spectral measurements.

In some embodiments, portable spectrophotometer 104 may take spectral measurements and generate leaf spectral data in visible and/or near-infrared wavelength ranges. For example, portable spectrophotometer 104 may generate leaf spectral data in a wavelength range from 400nm-2500nm. In other examples, portable spectrophotometer 104 may generate leaf spectral data in smaller (e.g., 400nm-2000nm) or larger (e.g., 350nm-2500nm) wavelength ranges. Smaller wavelength ranges may enable faster completion of the spectral measurements and/or require fewer resources for computing the petiole nutrient values. Larger wavelength ranges may enable determination of the petiole nutrient values with higher accuracy and/or detection of a larger number of nutrients. The wavelength range may be manually or automatically adjusted based on the nutrient values being measured. For example, operator 38 may manually adjust the wavelength range. In other examples, the wavelength range may be automatically adjusted, for example, by remote server 108.

Portable spectrophotometer 104 may take the spectral measurements at discrete wavelength intervals within the measured wavelength range. For example, portable spectrophotometer 104 may take the spectral measurements at 0.5nm wavelength intervals within the measured wavelength range. The wavelength intervals used during the measurements may be manually or automatically adjusted based on the nutrient values being determined. For example, operator 38 may manually adjust the wavelength interval. In other examples, the wavelength interval may be automatically adjusted, for example, by remote server 108. Different wavelength intervals ranging from 0.1nm to 10nm may be used. Smaller wavelength intervals may enable determination of nutrient values with higher accuracy and/or determination of a larger number of nutrients. Larger wavelength intervals may enable faster determination of nutrient values and/or require fewer computing resources for determination of the nutrient values.

In some embodiments, portable spectrophotometer 104 may be a NIRS^{™} DS2500 Analyzer or an ASD^{™} FieldSpec 4 spectroradiometer. In other embodiments, portable spectrophotometer 104 may be any other suitable spectrophotometer.

Portable spectrophotometer 104 may be configured to transmit the generated leaf spectral data for receipt by remote server 108. In the illustrated example, portable spectrophotometer 104 may transmit the generated leaf spectral data to remote server 108 via network 112. In other examples, portable spectrophotometer 104 may transmit the generated leaf spectral data to an intermediary device, for example, a mobile device 42 of operator 38. Portable spectrophotometer 104 may transmit the generated leaf spectral data to mobile device 42 using point-to-point communication or a local-area network (e.g. Bluetooth^{™}) and mobile device 42 may then transmit the leaf spectral data to remote server 108 using a large-area network/internet.

Mobile device 42 may be any suitable device that is portable and capable of sending and receiving data over a communication network (e.g., wireless communication networks like cellphone networks and/or wi-fi networks). For example, mobile device 42 may be a laptop, a tablet device, or a smartphone with wireless communication capabilities. Mobile device 42 may be a dedicated companion device designed for use with portable spectrophotometer 104. In some embodiments, mobile device 42 may be a general-purpose device that includes proprietary software for communication with portable spectrophotometer 104 and/or analysis of data received from portable spectrophotometer 104. In some embodiments, the functionality of portable spectrophotometer 104 and mobile device 42 may be provided by a single device.

In the illustrated example, remote server 108 includes a processor 116 and a memory 120. Processor 116 may control the operation of remote server 108. Processor 116 may be any suitable processor, controller or digital signal processor that can provide sufficient processing power depending on the configuration, purposes and requirements of system 100, as is known by those skilled in the art. For example, processor 116 may be a high-performance general processor. For example, processor 116 may include a standard processor, such as an Intel^{®} processor, or an AMD^{®} processor. Alternatively, processor 116 may include more than one processor with each processor being configured to perform different dedicated tasks. Alternatively, specialized hardware (e.g., graphical processing units (GPUs)) can be used provide some of the functions provided by processor 116.

Memory 120 may include one or more of random-access memory (RAM), read-only memory (ROM), a hard drive, and a flash memory (e.g. solid state drive). Remote server 108 may store received leaf spectral data in memory 120. For example, remote server 108 may receive leaf spectral data generated by portable spectrophotometer 104 and store the received leaf spectral data in memory 120.

Memory 120 may also store a machine learning (ML) model trained to provide petiole nutrient value outputs based on leaf spectral data inputs. The machine learning model may be generated and trained by remote server 108. In some embodiments, remote server 108 may receive a machine learning model generated by an external device. Remote server 108 may train the received machine learning model and store the trained machine learning model in memory 120. In other embodiments, the trained machine learning model may be received by remote server 108 from an external device. Remote server 108 may store the received trained machine learning model in memory 120.

In various embodiments, any one or more (or all) memories (e.g. memory 120) described herein as storing machine learning model(s) may store a machine learning pipeline that includes the machine learning model(s) in addition to associated data pre-processing code (e.g. for building, training, and/or evaluating the machine learning model(s)).

The leaf spectral data inputs provided to the machine learning model may include, for example, leaf spectral data generated by portable spectrophotometer 104 by taking spectral measurements from leaf blades of plants. The petiole nutrient value outputs provided by the machine learning model may include, for example, determined petiole nutrient values for one or more nutrients such as N, P, K, Ca, Mg, S, Mn, Zn, Fe, B, Cu, Al, and Na. The petiole nutrient values for the different nutrients may be expressed as percentage values (of total mass), g/kg, and/or parts per million (ppm).

The machine learning model may be any suitable machine learning model based on the input data to be analyzed, the output data requirements, the available training data and/or available computing resources. In some embodiments, the machine learning model may be composed of one or more univariate or multivariate multiple linear regression (MLR) models. Lasso or ridge regularization may be utilized in the MLR model to generate a lasso or ridge regression model. In other embodiments, the machine learning model may be a different model, for example, a polynomial regression model. In other embodiments, other data pre-processing steps may also be employed, such as dimensionality reduction through the Maximum Relevance - Minimum Redundancy (MRMR) method.

The training data for the machine learning model may be generated by taking spectral measurements of plants (e.g., reflectance spectral measurements from leaf blade portions) and generating leaf spectral data that act as predictors (x) for the machine learning model. Corresponding petioles of the plants may be removed and used for laboratory tissue analysis. The laboratory tissue analysis results of the petioles can act as responses (y) for the machine learning model. Models of correlation may be built between the responses (y) and the predictors (x). Equation (1) below, provides an example of a MLR model using correlation coefficients βᵢ: $y = β + {\sum }_{i = 1}^{{Z}_{i}} {x}_{i} {β}_{i}$

The number of predictors (x) being larger than the number of responses (y) can result in over-fitting of the MLR model. The accuracy of the MLR model may be improved by using subset selection methods to select a subset of the predictors. In some embodiments, lasso MLR modelling may be used as the subset selection method. In other embodiments, other subset selection methods like stepwise subset selection or Bayesian model averaging may be used.

Lasso MLR modelling may be used to identify the most informative, least redundant predictors (wavelengths) using a complexity parameter (λ) that controls the amount of shrinkage: the larger that the value of λ is, the greater can be the penalization of the non-zero coefficients in the model, and consequently a greater shrinkage can be imposed on coefficient values. A lasso regression model may be generated by selecting the value of λ that minimizes the root mean squared error (RMSE). The chosen λ parameter can determine the number of coefficients that will compose the lasso regression model, which are selected as the ones with the greatest explanatory power in relation to the target variable to be predicted. Model training and performance assessment may be conducted, for example, using K-fold cross validation using different integer values of K such as 5, with the value of λ chosen based on the smallest RMSE. Table 1 below shows values for number, range, and first four significant wavebands resulting from lasso MLR modelling of different elements for example leaf spectral data obtained from potato plants.

**Table 1 Values for number, range, and first four significant wavebands resulting from lasso MLR modelling of different elements for example leaf spectral data obtained from potato plants.**

| Element | Number of bands | Range of bands (nm) | First four significant wavebands (nm) |
|---|---|---|---|
| N | 13 | 404-1828 | 660, 684, 404, 484 |
| P | 10 | 404-1924 | 708, 404, 540, 700 |
| K | 17 | 404-2300 | 404, 428, 588, 948 |
| Ca | 20 | 404-2100 | 404, 444, 588, 540 |
| Mg | 14 | 404-1940 | 700, 532, 1716, 524 |
| S | 18 | 404-1916 | 404, 588, 516, 1452 |
| Mn | 22 | 428-2492 | 660, 628, 428, 492 |
| Zn | 12 | 468-2124 | 1932, 524, 1852, 532 |
| Fe | 19 | 404-2316 | 1932, 636, 524, 2308 |
| B | 11 | 412-1932 | 684, 1932, 412, 460 |
| Cu | 23 | 428-2484 | 1940, 676, 428, 1716 |
| Al | 17 | 404-2316 | 1932, 2308, 524, 652 |
| Na | 20 | 548-2148 | 548, 972, 700, 1028 |

Processor 116 may input the received leaf spectral data (e.g., from portable spectrophotometer 104) and/or stored leaf spectral data (e.g., in memory 120) into the trained machine learning model. The machine learning model can estimate petiole nutrient values based on the leaf spectral data. For example, the machine learning model can estimate petiole nutrient values for N, P, K, Ca, Mg, S, Mn, Zn, Fe, B, Cu, Al, and Na based on leaf spectral data generated by taking spectral measurements of leaf blades of potato plants. Processor 116 may receive the petiole nutrient values determined by the machine learning model.

Processor 116 can provide a near real-time result indicating the petiole nutrient values to a mobile device. For example, processor 116 may provide the near real-time result to mobile device 42 of operator 38. For example, processor 116 may provide the near real-time results to mobile device 42 less than 1 minute (e.g., within 0.01 to 30 seconds) from when the portable spectrophotometer sent the spectral measurements. This may enable operator 38 to non-destructively collect near real-time results indicating petiole nutrient values from multiple plants in a crop field.

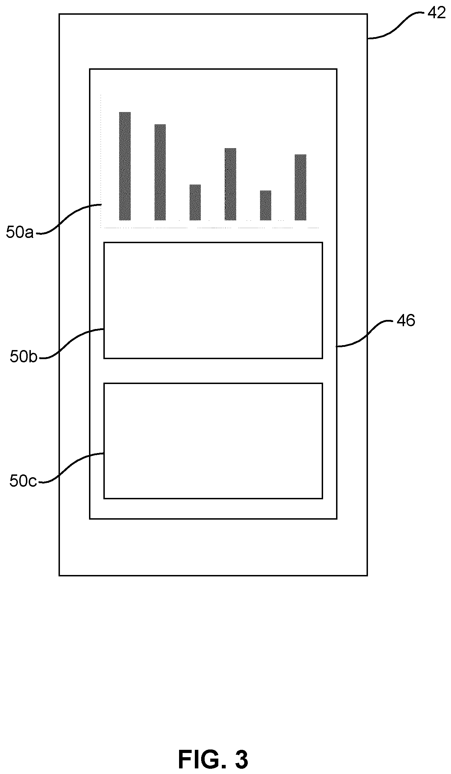

Reference is now made to FIGS. 2 and 3. FIG. 3, shows a schematic illustration of a result indicating petiole nutrient values provided by system 100 and graphically displayed on mobile device 42. Mobile device 42 may include a touchscreen display 46 that provides an interactive display to operator 38. Touchscreen display 46 may provide multiple display portions 50a-50c. For example, display portion 50a may provide a graphical display of the determined petiole nutrient values. In the illustrated example, display portion 50a provides bar graphs displaying determined petiole nutrient values for six nutrients. In other embodiments, display 46 may not be configured to detect touch inputs.

Display portion 50b may provide additional information regarding the determined petiole nutrient values, for example, number of leaf spectral measurements taken, confidence levels associated with the determined petiole nutrient values etc. Display portion 50c may provide recommended corrective actions based on the determined petiole nutrient values. In some examples, one or more of display portions 50 may include other information unrelated to the results indicating the petiole nutrient values.

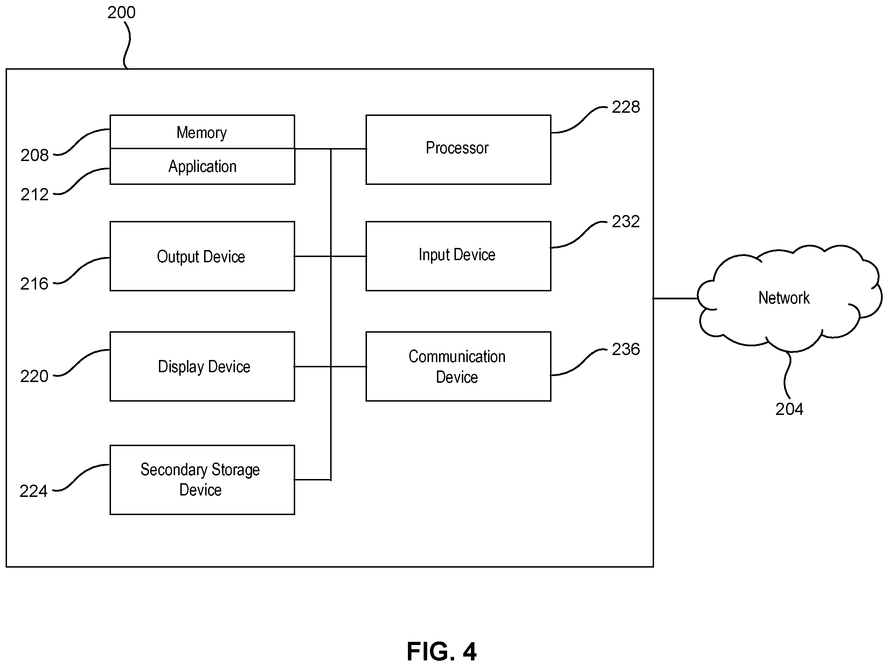

Referring now to FIG. 4, shown therein is a schematic illustration of an apparatus 200 for non-destructively determining petiole nutrient values in near real-time from plant leaves in a crop field. In some embodiments, apparatus 200 may be implemented as a server device (e.g., remote server 108 shown in FIG. 2). In other embodiments, apparatus 200 may be implemented using any other suitable hardware components. For example, apparatus 200 may be implemented as mobile device 42 (FIGS. 2 and 3), integrated with portable spectrophotometer 104 (FIG. 2), or implemented as a companion apparatus usable with portable spectrophotometer 104 (FIG. 2).

For the example embodiment illustrated in FIG. 4, apparatus 200 includes a memory 208, an application 212, an output device 216, a display device 220, a secondary storage device 224, a processor 228, an input device 232, and a communication device 236. One or more (or all) of memory 208, application 212, output device 216, display device 220, secondary storage device 224, processor 228, input device 232, and communication device 236 may be communicatively coupled by wire and/or wirelessly.

In some embodiments, apparatus 200 includes multiple of any one or more of memory 208, application 212, output device 216, display device 220, secondary storage device 224, processor 228, input device 232, and communication device 236. In some embodiments, apparatus 200 does not include one or more of applications 212, secondary storage devices 224, network connections, input devices 232, output devices 216, display devices 220, and communication devices 236.

In at least one embodiment, apparatus 200 includes a connection with a network 204 such as a wired or wireless connection to the Internet or to a private network. In some cases, network 204 includes other types of computer or telecommunication networks. Apparatus 200 may receive, over network 204, leaf spectra data generated by a portable spectrophotometer (e.g., portable spectrophotometer 104 of FIG. 2) based on non-destructive spectral measurements of a leaf of a plant (e.g., a potato plant) in the crop field (e.g., crop field 34 of FIG. 2).

In some embodiments, apparatus 200 may receive the leaf spectral data directly from the portable spectrophotometer by wire or wirelessly. In other embodiments, apparatus 200 may receive the leaf spectral data generated by the portable spectrophotometer from an intermediary device (e.g., mobile device 42 of FIG. 2).

Memory 208 can include one or more of random-access memory (RAM) and read-only memory (ROM). In some embodiments, memory 208 stores one or more applications 212 for execution by processor 228. Applications 212 correspond with software modules including computer executable instructions to perform processing for the functions and methods described herein.

Memory 208 may store a machine learning model trained to provide petiole nutrient value outputs based on leaf spectral data inputs. The machine learning model may be generated and trained by apparatus 200. In some embodiments, apparatus 200 may receive a generated machine learning model from an external device (e.g., over network 204) and may train the received machine learning model. In some embodiments, apparatus 200 may receive a trained machine learning model from an external device (e.g., over network 204) and store the trained machine learning model in memory 208. The machine learning model may be any suitable model, e.g., a lasso regression model.

In some embodiments, memory 208 may also store received leaf spectral data, determined petiole nutrient values, and/or results derived from the petiole nutrient values (e.g. a graphical representation of the petiole nutrient values). The stored data may be retrieved by a user for historical data analysis.

Secondary storage device 224 may include any suitable non-transitory computer-readable medium including instructions executable by a processor (e.g., processor 228). For example, secondary storage device 224 can include a hard drive, floppy drive, CD drive, DVD drive, Blu-ray drive, solid state drive, flash memory or other types of nonvolatile data storage. Processor 228 may execute instructions included on secondary storage device 224 to perform processing for the functions and methods described herein.

In some embodiments, apparatus 200 stores information in a remote storage device, such as cloud storage, accessible across a network, such as network 204 or another network. In some embodiments, apparatus 200 stores information distributed across multiple storage devices, such as memory 208 and secondary storage device 224 (i.e., each of the multiple storage devices stores a portion of the information and collectively the multiple storage devices store all of the information). Accordingly, storing data on a storage device as used herein and in the claims, means storing that data in a local storage device, storing that data in a remote storage device, or storing that data distributed across multiple storage devices, each of which can be local or remote.

Input device 232 can include any device for entering information into apparatus 200. For example, input device 232 can be a keyboard, keypad, cursor-device, touchscreen, camera, or microphone. Input device 232 can also include input ports and wireless radios (e.g., Bluetooth^{®}, or 802.11x) for making wired and wireless connections to external devices.

Display device 220 can include any type of device for presenting visual information. For example, display device 220 can be a computer monitor, a flat-screen display, a projector or a display panel.

Output device 216 can include any type of device for presenting a hard copy of information, such as a printer for example. Output device 216 can also include other types of output devices such as speakers, for example. In at least one embodiment, output device 216 includes one or more of output ports and wireless radios (e.g., Bluetooth^{®}, or 802.11x) for making wired and wireless connections to external devices.

Communication device 236 can have any design suitable to receive analog and/or digital inputs from, and to provide analog and/or digital outputs. In some embodiments, communication device 236 may include separate modules for analog and digital signals.

Processor 228 may be any device that can execute applications, computer readable instructions or programs. The applications, computer readable instructions or programs can be stored in memory 208 or in secondary storage device 224, or can be received from remote storage accessible through network 204, for example. Processor 228 may be a high-performance general processor, a standard processor (e.g., an Intel^{®} processor or an AMD^{®} processor), specialized hardware (e.g., GPUs), or multiple processing devices that collectively perform the functions provided by processor 228.

Processor 228 may input leaf spectral data (e.g., leaf spectral data stored in memory 208) into a trained machine learning model (e.g., trained machine learning model stored in memory 208). The machine learning model can determine petiole nutrient values based on the leaf spectral data. Processor 228 may receive an output from the machine learning model that includes the petiole nutrient values determined by the machine learning model.

In some embodiments, processor 228 can provide a near real-time result indicating the petiole nutrient values to a mobile device (e.g., mobile device 42 of FIG. 2). The petiole nutrient values may be graphically displayed on the mobile device. In other embodiments, processor 228 may provide the results to display device 220 and the petiole nutrient values may be graphically displayed on display device 220.

FIG. 4 illustrates one example hardware schematic of an apparatus 200. In alternative embodiments, apparatus 200 contains fewer, additional or different components. In addition, although aspects of an implementation of apparatus 200 are described as being stored in memory, one skilled in the art will appreciate that these aspects can also be stored on or read from other types of computer program products or computer-readable media, such as secondary storage devices, including hard disks, floppy disks, CDs, or DVDs; a carrier wave from the Internet or other network; or other forms of RAM or ROM. For example, apparatus 200 may include a non-transitory computer readable medium storing computer-readable instructions that when executed by processor 228, configure processor 228 to perform method(s) described herein.

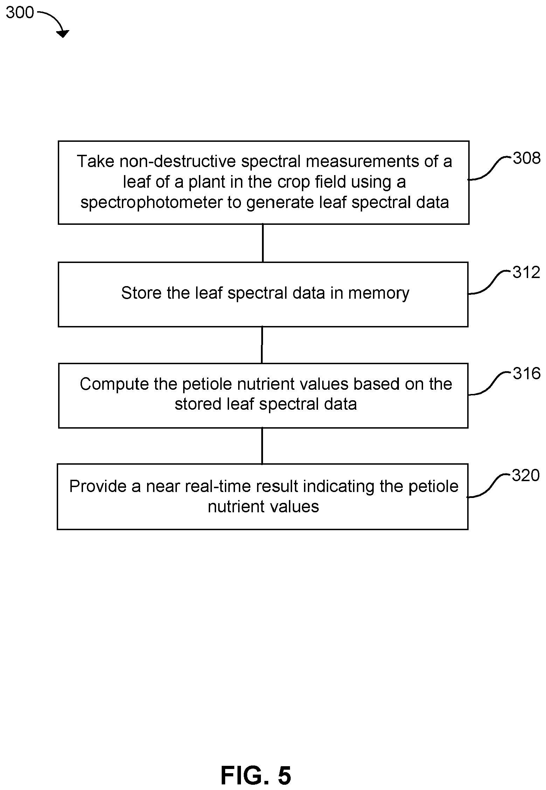

Referring now to FIG. 5, shown therein is a flowchart illustrating an example method 300 of non-destructively determining petiole nutrient values in near real-time from plant leaves in a crop field using a portable spectrophotometer. Method 300 may be performed, for example, using system 100 (FIG. 2) or apparatus 200 (FIG. 4) and reference is also made below to FIGS. 2 and 4.

Method 300 may be performed while carrying the portable spectrophotometer in the crop field. For example, portable spectrophotometer 104 may be carried in crop field 34 on a person or vehicle.

At 308, non-destructive spectral measurements may be taken of a leaf of a plant in the crop field using the carried spectrophotometer to generate leaf spectral data. For example, non-destructive spectral measurements may be taken of a leaf of a potato plant using portable spectrophotometer 104. Portable spectrophotometer 104 can generate leaf spectral data based on the spectral measurements.

At 312, the leaf spectral data may be stored in a memory. In some embodiments, the generated leaf spectral data may be sent to a remote server over a network and stored in a memory of the remote server. For example, portable spectrophotometer 104 may send the generated leaf spectral data to remote server 108 over network 112 and remote server 108 may store the leaf spectral data in memory 120.

In other embodiments, the generated leaf spectral data may be sent to a mobile device and the mobile device may send the leaf spectral data to a remote server over a network. The leaf spectral data may be stored in a memory of the remote server. For example, portable spectrophotometer 104 may send the generated leaf spectral data to mobile device 42 and mobile device 42 may send the leaf spectral data to remote server 108 over network 112. Remote server 108 may store the leaf spectral data in memory 120.

At 316, a processor may compute the petiole nutrient values based on the stored leaf spectral data. The processor may compute the petiole nutrient values by inputting the leaf spectral data into a machine learning model trained to provide petiole nutrient value outputs based on leaf spectral data inputs. In response, the machine learning model may output the petiole nutrient values to the processor. For example, processor 116 (or processor 228) may input the leaf spectral data into a trained machine learning model stored in memory 120 (or memory 208). The machine learning model may be a lasso regression model.

At 320, a near real-time result indicating the petiole nutrient values may be provided. In some embodiments, the near real-time result may be provided by sending the petiole nutrient values to a mobile device. The petiole nutrient values may then be graphically displayed on the mobile device. For example, remote server 108 or apparatus 200 may provide the near real-time result by sending the petiole nutrient values to mobile device 42. Mobile device 42 may graphically display the petiole nutrient values.

In some embodiments, method 300 may proceed to 308 if there are additional leaves for measurement. For example, method 300 may loop through 308 to 320 for at least five other plant leaves in the crop field within ten minutes. This may enable near real-time results to be obtained indicating the petiole nutrient values corresponding to non-destructive spectral measurements from multiple leaves in a short time period. In turn this will allow the farm to contemporaneously react to the returned petiole nutrient values (e.g., by adjusting the composition and/or quantity of fertilizer) before the nutrient content of the measured plants has had time to change. This may improve the effectiveness of the corrective actions, whereby the crop yield and/or quantity may be improved.

In the invention, processing the leaf spectral data with machine learning follows a two-layered approach to provide more accurate estimates of petiole nutrient values (referred to herein as "refined petiole nutrient values"). A first ML layer involves one or more first machine learning models (e.g., one or more independent instances of the machine learning model described above with reference to FIGS. 1-5). A second ML layer involves one or more second machine learning models. The petiole nutrient values determined by the first machine learning model(s) in the first layer is refined to produce statistically more accurate values (i.e., values for one or more petiole nutrients that are closer to the actual values of the one or more nutrients of a petiole that would have been obtained had the petiole been removed and destructively measured at a lab according to traditional methods). In the invention, the petiole nutrient values determined by the first layer of machine learning model(s) are referred to as preliminary petiole nutrient values.

The first layer of machine learning model(s) may be trained and stored in the manner described previously. In some embodiments, only a small subset of the most explanatory features (i.e. intensity values related to specific wavelengths or wavelength intervals of the collected leaf spectral data) are input into the first machine learning model(s). This addresses the problem of the dataset including a large number of features (i.e., the high dimensionality of the feature space). Before feeding the features into the first layer of machine learning model(s), a feature reduction step may be performed (e.g., minimum redundancy - maximum relevance (MRMR) feature selection) independently for each target variable to extract a subset of features to be input into the first layer of machine learning model(s). Pre-selecting features can improve the estimation performance of most nutrients. A model regularization step may also be performed, e.g., a lasso regression model, as explained above, which removes any features not deemed to contribute significantly to the predictive ability of the model.

Memory 120 (or memory 208) may store the second layer of machine learning model(s) trained to provide refined petiole nutrient value outputs based on preliminary petiole nutrient values output by the first layer of machine learning model(s). The second layer of machine learning model(s) may be generated and trained by remote server 108. In some embodiments, remote server 108 may receive a second layer of machine learning model(s) generated by an external device. Remote server 108 may train the received second layer of machine learning model(s) and store the trained second layer of machine learning model(s) in memory 120 (or memory 208). In other embodiments, the trained second layer of machine learning model(s) may be received by remote server 108 from an external device. Remote server 108 may store the received trained second layer of machine learning model(s) in memory 120 (or memory 208).

The second layer of machine learning model(s) is composed of any suitable non-linear machine learning model based on the input data to be analyzed, the output data requirements, the available training data and/or available computing resources. In some embodiments, one or more (or all) of the second machine learning model(s) used in the second layer may be non-linear multiple regression models, such as but not limited to Support Vector Regression (SVR), Random Forest (RF) or Extreme Gradient Boosting (XGB).

As described above, the first machine learning model(s) in the first layer may be trained using sets of data that include matching sets of leaf spectral data and lab-measured petiole nutrient values. This allows the first layer of machine learning model(s) to establish relationships between the leaf spectral data values and the individual petiole nutrient values.

Generally, there is overlapping influence on the reflectance at different wavelengths due to intricate relationships between nutrient concentrations in plants. A certain species of plant will have a certain propensity to uptake a variety of nutrients (i.e., a species has specific nutrient uptake characteristics). The uptake of certain nutrients will be impacted by the uptake of other nutrients. For example, for one species of plant, when the plant uptakes a certain nutrient "x", it will tend to also uptake nutrient "y" (i.e. there is a positive correlation between nutrient "x" and nutrient "y"). But when the plant uptakes nutrient "x", it may also tend to reduce the uptake of nutrient "z" (i.e., there is a negative correlation between nutrient "x" and nutrient "z"). For instance, potassium (K) and magnesium (Mg) are known for their antagonisms as competitive cations for plant uptake.

Therefore, refining preliminary petiole nutrient values determined from leaf spectral data based on the interlinkages among the nutrients for the given plant species may provide more statistically accurate results.

The non-linearity of the second machine learning model(s) in the second layer allows the model(s) to account for these nutrient uptake characteristics and potentially correlated outcomes simultaneously. The second machine learning model(s) in the second layer may be trained using sets of data that include a large number of different petiole nutrient values. The second machine learning model(s) in the second layer processes this data to establish the uptake relationships between different nutrients for a given plant species. Importantly, the nutrient uptake characteristics for different species of plants will vary.

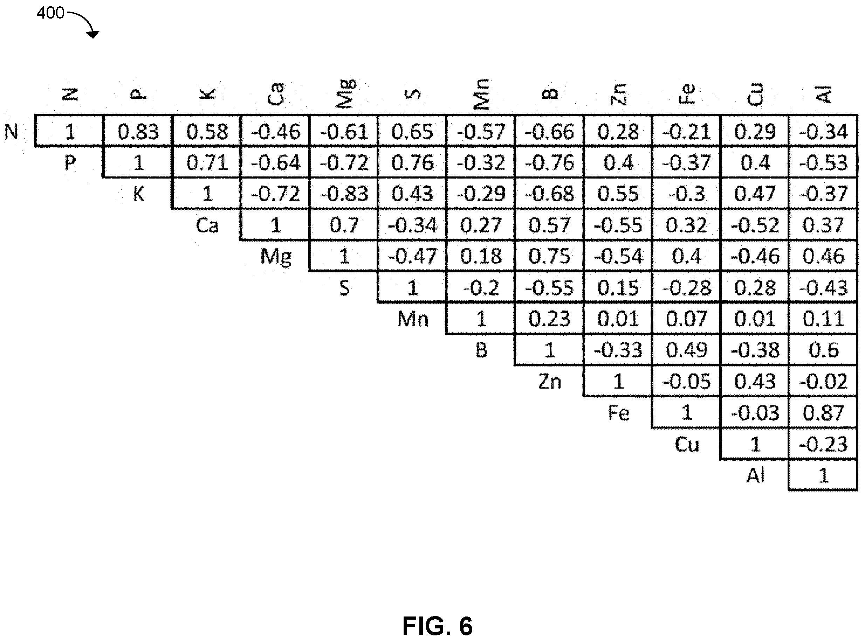

FIG. 6 shows a table 400 which demonstrates an example nutrient uptake characteristic for a species of potato plants. As can be seen in FIG. 6, for example, the potato plant's uptakes of nitrogen (N) tends to promote the uptake of phosphorus (P) (shown with the positive value 0.83). However, when a potato plant uptakes nitrogen, it also tends to reduce the uptake of boron (B) (shown with negative value -0.66). In other words, there is a positive correlation between the uptake of nitrogen and phosphorus, but a negative correlation between the uptake of nitrogen and boron. As shown in FIG. 6, a correlation may be determined between each expected petiole nutrient. Accounting for correlations between the uptake of different nutrients allows for the refined petiole nutrient values to be statistically more accurate than the preliminary petiole nutrient values output from the first machine learning model(s) of the first layer to which leaf spectral data was input.

The reasons for the positive and negative correlations in the uptake of different nutrients are widely varied. The following illustrate a few examples which may apply to one or more species of potato plants. The positive relationship between N and P may be because N fixation can depend on P availability in the soil for plant uptake. The counteracting effect between calcium (Ca) and P may be caused by the precipitation of less soluble calcium phosphates in the vicinity of nutrient-absorbing roots. K and Mg may be competitive cations for plant uptake. Ca and Mg may have synergic contribution to maintaining a stable pH in soil. N may be negatively interlinked with Mg as the increase in N rates can be reduced by the addition of Mg rates. B has a negative correlation with P, but B availability is positively influenced by the presence of Ca. The plant uptake of K may be attributed to N for its role in leaf area development and photosynthesis. The form of K application as sulfate can lead to the assimilation distribution of sulfur (S). For example, some K fertilizer includes potassium as K₂SO₄ such that the application of potassium is accompanied by sulfur. An increase in K supply (e.g. to the ground by fertilizer) typically results in an increase in K uptake by the plant and this can decrease the concentration of manganese (Mn) in the leaves. An increasing supply of S (e.g. to the ground by fertilizer) typically results in an increase in S uptake by the plant and this may improve the absorption of K and P. A high dose of K may decrease the uptake of iron (Fe) as the tolerance to Fe toxicity improves. There is a countereffect between copper (Cu) and Ca as Ca translocation from roots to leaves may be disrupted in the presence of Cu.

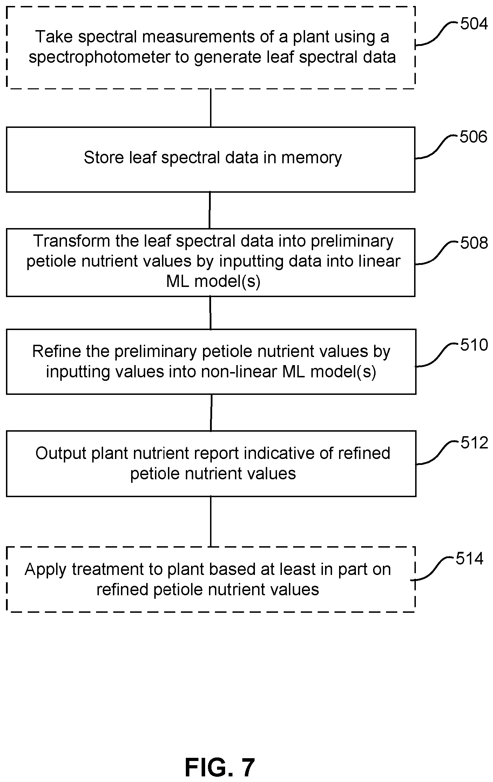

Referring now to FIG. 7, shown therein is a flowchart illustrating an example method 500 of determining refined petiole nutrient values based on leaf spectral data from plant leaves, as in the present invention. Method 500 may be performed, for example, using system 100 (FIG. 2) or apparatus 200 (FIG. 4) and reference is also made below to FIGS. 2 and 4.

Method 500 may be performed while carrying the portable spectrophotometer in the crop field. For example, portable spectrophotometer 104 may be carried in crop field 34 on a person or vehicle. Method 500 may also be performed using any other suitable spectrophotometer, including spectrophotometers that are not portable.

The method 500 may optionally include step 504. At 504, spectral measurements may be taken of a leaf of a plant using a spectrophotometer (e.g., portable spectrophotometer 104) to generate leaf spectral data. For example, the spectral measurements may be taken of a potato plant leaf.

At 506, the leaf spectral data are stored in a memory. In some embodiments, the generated leaf spectral data may be sent to a remote server over a network and stored in a memory of the remote server. For example, portable spectrophotometer 104 may send the generated leaf spectral data to remote server 108 over network 112 and remote server 108 may store the leaf spectral data in memory 120.

In other embodiments, the generated leaf spectral data may be sent to a mobile device and the mobile device may send the leaf spectral data to a remote server over a network. The leaf spectral data may be stored in a memory of the remote server. For example, portable spectrophotometer 104 may send the generated leaf spectral data to mobile device 42 and mobile device 42 may send the leaf spectral data to remote server 108 over network 112. Remote server 108 may store the leaf spectral data in memory 120.

At 508, a processor transforms the leaf spectral data into preliminary petiole nutrient values. The processor transforms the leaf spectral data by inputting the leaf spectral data into one or more linear machine learning models trained to provide petiole nutrient value outputs based on leaf spectral data inputs. In response, the linear machine learning model(s) outputs the preliminary petiole nutrient values to the processor. For example, processor 116 (or processor 228) may input the leaf spectral data into one or more trained linear machine learning models stored in memory 120 (or memory 208). One or more (or all) of the linear machine learning model(s) may be a lasso regression model.

At 510, the processor refines the preliminary petiole nutrient values by inputting the preliminary petiole nutrient values into one or more non-linear machine learning models trained to provide refined petiole nutrient value outputs based on preliminary petiole nutrient value inputs. In response, the non-linear machine learning model(s) outputs the refined petiole nutrient values to the processor. For example, processor 116 (or processor 228) may input the preliminary petiole nutrient values into trained non-linear machine learning model(s) stored in memory 120 (or memory 208). One or more (or all) of the non-linear machine learning model(s) may be a multivariate regression model.

At 512, a plant nutrient report indicative of the refined petiole nutrient values is outputted. A plant nutrient report may have any suitable format, including but not limited to a list, a table, a chart, a graphical representation, any type of visualization (e.g., infographic, map, etc.), or an animation of the refined petiole nutrient values. In some embodiments, the plant nutrient report may be outputted by sending the refined petiole nutrient values to a mobile device. For example, remote server 108 or apparatus 200 may provide the near real-time result (e.g. in less than 1 minute from the completion of step 506) by sending the refined petiole nutrient values to mobile device 42. Mobile device 42 may graphically display the petiole nutrient values.

In some embodiments, method 500 may proceed to step 504 or 506 if there are additional leaves for measurement. For example, method 500 may loop through 504 and/or 506 to 512 for at least five other plant leaves in the crop field within approximately one hour. This may enable results to be obtained indicating the refined petiole nutrient values corresponding to spectral measurements from multiple leaves in a short time period. In turn this will allow the farm to contemporaneously react to the returned refined petiole nutrient values (e.g., by adjusting the composition and/or quantity of fertilizer) before the nutrient content of the measured plants has had time to change. This may improve the effectiveness of the corrective actions, whereby the crop yield and/or quantity may be improved.

At 514, a treatment may optionally be applied to the crop (e.g. including and/or in the vicinity of the measured plants). The treatment may include applying (e.g. spraying or spreading) liquid and/or solid (e.g. granular) fertilizer to the crop. The treatment may be determined (e.g. by processor 116 or 228) based at least in part on the refined nutrient values of step 512. The treatment may be applied under the control of a human operator and/or as directed by electronic automation (e.g. directed by processor 116 or 228).

The two-layered approach of the invention, which includes linear and non-linear machine learning models, can increase the accuracy of nutrient prediction substantially. In particular, the estimation of Zn, Fe and Al is substantially more accurate compared to using solely linear machine learning model(s) in a one layer approach.

While the above description provides examples of the embodiments, it will be appreciated that some features and/or functions of the described embodiments are susceptible to modification**.** Accordingly, what has been described above has been intended to be illustrative of the invention and non-limiting and it will be understood by persons skilled in the art that other variants and modifications may be made without departing from the scope of the invention as defined in the claims appended hereto.

## Claims

1. A method (500) of determining refined petiole nutrient values based on leaf spectral data from plant leaves (18), the method comprising:
(a) storing (506) the leaf spectral data in a memory (120);
(b) transforming (508), using a processor (116), the leaf spectral data into preliminary petiole nutrient values by inputting the leaf spectral data into one or more linear machine learning models trained to provide petiole nutrient value outputs based on leaf spectral data inputs, the one or more linear machine learning models outputting the preliminary petiole nutrient values;
**characterized by**:
(c) refining (510), using the processor, the preliminary petiole nutrient values by inputting the preliminary petiole nutrient values into one or more non-linear machine learning models trained to provide refined petiole nutrient value outputs based on preliminary petiole nutrient value inputs, the one or more non-linear machine learning models outputting refined petiole nutrient values; and
(d) outputting (512) a plant nutrient report indicative of the refined petiole nutrient values,
wherein the one or more non-linear machine learning models are trained to model interrelations in the nutrient uptake characteristics of different plant nutrients.

2. The method (500) of claim 1, further comprising, prior to said storing, taking (504) spectral measurements of a leaf (18) of a plant (10) using a spectrophotometer (104) to generate leaf spectral data.

3. The method (500) of any preceding claim, wherein each of the one or more non-linear machine learning models is a multivariate regression model.

4. The method (500) of any preceding claim, further comprising applying (514) a fertilizer treatment to plants based at least in part on the refined petiole nutrient values.

5. A non-transitory computer-readable medium comprising instructions executable by a processor (116), wherein the instructions when executed configure the processor to:
store (506) leaf spectral data from plant leaves (18) in a memory (120);
transform (508), using the processor, the leaf spectral data into preliminary petiole nutrient values by inputting the leaf spectral data into one or more linear machine learning models trained to provide petiole nutrient value outputs based on leaf spectral data inputs;
receive an output comprising the preliminary petiole nutrient values from the one or more linear machine learning models;
refine (510), using the processor, the preliminary petiole nutrient values by inputting the preliminary petiole nutrient values into one or more non-linear machine learning models trained to provide refined petiole nutrient value outputs based on preliminary petiole nutrient value inputs;
receive an output comprising the refined petiole nutrient values from the one or more non-linear machine learning models; and
output (512) a plant nutrient report indicative of the refined petiole nutrient values,
wherein the one or more non-linear machine learning models are trained to model interrelations in the nutrient uptake characteristics of different plant nutrients.

6. The non-transitory computer-readable medium of claim 5, wherein the instructions when executed configure the processor to receive the spectral data taken of a leaf (18) of a plant (10) using a spectrophotometer (104).

7. The non-transitory computer-readable medium of any one of claims 5-6, wherein each of the one or more non-linear machine learning models is a multivariate regression model.

8. The non-transitory computer-readable medium of any one of claim 5-7, wherein the instructions when executed configure the processor to apply (514) a fertilizer treatment to plants based at least in part on the refined petiole nutrient values.

9. A system for determining refined petiole nutrient values based on leaf spectral data from plant leaves (18), the system comprising:
a memory (120) configured to store leaf spectral data from plant leaves (18); and
a processor (116) configured to:
transform (508) the leaf spectral data into preliminary petiole nutrient values by inputting the leaf spectral data into one or more linear machine learning models trained to provide petiole nutrient value outputs based on leaf spectral data inputs;
receive an output comprising the preliminary petiole nutrient values from the one or more linear machine learning models;
**characterized in that** the processor is further configured to:
refine (510) the preliminary petiole nutrient values by inputting the preliminary petiole nutrient values into one or more non-linear machine learning models trained to provide refined petiole nutrient value outputs based on preliminary petiole nutrient value inputs;
receive an output comprising the refined petiole nutrient values from the one or more non-linear machine learning models; and
output (512) a plant nutrient report indicative of the refined petiole nutrient values,
wherein the one or more non-linear machine learning models are trained to model interrelations in the nutrient uptake characteristics of different plant nutrients.

10. The system of claim 9, wherein the processor is further configured to receive the spectral data taken of a leaf (18) of a plant (10) using a spectrophotometer (104).

11. The system of any one of claims 9-10, wherein each of the one or more non-linear machine learning models is a multivariate regression model.

12. The system of any one of claims 9-11, wherein the processor is further configured to apply (514) a fertilizer treatment to plants based at least in part on the refined petiole nutrient values.

## Patentansprüche

1. Verfahren (500) zur Bestimmung von verfeinerten Blattstielnährstoffwerten auf Basis von Blattspektraldaten von Pflanzenblättern (18), wobei das Verfahren Folgendes umfasst:
(a) Speichern (506) der Blattspektraldaten in einem Speicher (120);
(b) Umwandeln (508), unter Verwendung eines Prozessors (116), der Blattspektraldaten in vorläufige Blattstielnährstoffwerte durch Eingeben der Blattspektraldaten in ein oder mehrere lineare Maschinenlernmodelle, die dazu trainiert sind, Blattstielnährstoffwertausgaben auf Basis von Blattspektraldateneingaben bereitzustellen, wobei das eine oder die mehreren linearen Maschinenlernmodelle die vorläufigen Blattstielnährstoffwerte ausgeben;
**gekennzeichnet durch**:
(c) Verfeinern (510), unter Verwendung des Prozessors, der vorläufigen Blattstielnährstoffwerte durch Eingeben der vorläufigen Blattstielnährstoffwerte in ein oder mehrere nichtlineare Maschinenlernmodelle, die dazu trainiert sind, verfeinerte Blattstielnährstoffwertausgaben auf Basis von vorläufigen Blattstielnährstoffwerteingaben bereitzustellen, wobei das eine oder die mehreren nichtlinearen Maschinenlernmodelle verfeinerte Blattstielnährstoffwerte ausgeben; und
(d) Ausgeben (512) eines Pflanzennährstoffberichts, der die verfeinerten Blattstielnährstoffwerte angibt,
wobei das eine oder die mehreren nichtlinearen Maschinenlernmodelle dazu trainiert sind, Zusammenhänge in den Nährstoffaufnahmeeigenschaften von unterschiedlichen Pflanzennährstoffen zu modellieren.

2. Verfahren (500) nach Anspruch 1, ferner umfassend, vor dem Speichern, Vornehmen (504) von Spektralmessungen eines Blattes (18) einer Pflanze (10) unter Verwendung eines Spektrophotometers (104), um Blattspektraldaten zu erzeugen.

3. Verfahren (500) nach einem vorhergehenden Anspruch, wobei jedes von dem einen oder den mehreren nichtlinearen Maschinenlernmodellen ein multivariates Regressionsmodell ist.

4. Verfahren (500) nach einem vorhergehenden Anspruch, ferner umfassend Anwenden (514) einer Düngemittelbehandlung auf Pflanzen zumindest teilweise auf Basis der verfeinerten Blattstielnährstoffwerte.

5. Nichtflüchtiges computerlesbares Medium, das Anweisungen umfasst, die durch einen Prozessor (116) ausführbar sind, wobei die Anweisungen, wenn ausgeführt, den Prozessor zu Folgendem konfigurieren:
Speichern (506) von Blattspektraldaten von Pflanzenblättern (18) in einem Speicher (120);
Umwandeln (508), unter Verwendung des Prozessors, der Blattspektraldaten in vorläufige Blattstielnährstoffwerte durch Eingeben der Blattspektraldaten in ein oder mehrere lineare Maschinenlernmodelle, die dazu trainiert sind, Blattstielnährstoffwertausgaben auf Basis von Blattspektraldateneingaben bereitzustellen;
Empfangen einer Ausgabe, welche die vorläufigen Blattstielnährstoffwerte umfasst, von dem einen oder den mehreren linearen Maschinenlernmodellen;
Verfeinern (510), unter Verwendung des Prozessors, der vorläufigen Blattstielnährstoffwerte durch Eingeben der vorläufigen Blattstielnährstoffwerte in ein oder mehrere nichtlineare Maschinenlernmodelle, die dazu trainiert sind, verfeinerte Blattstielnährstoffwertausgaben auf Basis von vorläufigen Blattstielnährstoffwerteingaben bereitzustellen;
Empfangen einer Ausgabe, welche die verfeinerten Blattstielnährstoffwerte umfasst, von dem einen oder den mehreren nichtlinearen Maschinenlernmodellen; und
Ausgeben (512) eines Pflanzennährstoffberichts, der die verfeinerten Blattstielnährstoffwerte angibt,
wobei das eine oder die mehreren nichtlinearen Maschinenlernmodelle dazu trainiert sind, Zusammenhänge in den Nährstoffaufnahmeeigenschaften von unterschiedlichen Pflanzennährstoffen zu modellieren.

6. Nichtflüchtiges computerlesbares Medium nach Anspruch 5, wobei die Anweisungen, wenn ausgeführt, den Prozessor dazu konfigurieren, die Spektraldaten zu empfangen, die von einem Blatt (18) einer Pflanze (10) unter Verwendung eines Spektrophotometers (104) genommen werden.

7. Nichtflüchtiges computerlesbares Medium nach einem der Ansprüche 5-6, wobei jedes von dem einen oder den mehreren nichtlinearen Maschinenlernmodellen ein multivariates Regressionsmodell ist.

8. Nichtflüchtiges computerlesbares Medium nach einem der Ansprüche 5-7, wobei die Anweisungen, wenn ausgeführt, den Prozessor dazu konfigurieren, eine Düngemittelbehandlung auf Pflanzen zumindest teilweise auf Basis der verfeinerten Blattstielnährstoffwerte anzuwenden (514).

9. System zur Bestimmung von verfeinerten Blattstielnährstoffwerten auf Basis von Blattspektraldaten von Pflanzenblättern (18), wobei das System Folgendes umfasst:
einen Speicher (120), der dazu konfiguriert ist, Blattspektraldaten von Pflanzenblättern (18) zu speichern; und
einen Prozessor (116), der zu Folgendem konfiguriert ist:
Umwandeln (508) der Blattspektraldaten in vorläufige Blattstielnährstoffwerte durch Eingeben der Blattspektraldaten in ein oder mehrere lineare Maschinenlernmodelle, die dazu trainiert sind, Blattstielnährstoffwertausgaben auf Basis von Blattspektraldateneingaben bereitzustellen;
Empfangen einer Ausgabe, welche die vorläufigen Blattstielnährstoffwerte umfasst, von dem einen oder den mehreren linearen Maschinenlernmodellen;
**dadurch gekennzeichnet, dass** der Prozessor ferner zu Folgendem konfiguriert ist:
Verfeinern (510) der vorläufigen Blattstielnährstoffwerte durch Eingeben der vorläufigen Blattstielnährstoffwerte in ein oder mehrere nichtlineare Maschinenlernmodelle, die dazu trainiert sind, verfeinerte Blattstielnährstoffwertausgaben auf Basis von vorläufigen Blattstielnährstoffwerteingaben bereitzustellen;
Empfangen einer Ausgabe, welche die verfeinerten Blattstielnährstoffwerte umfasst, von dem einen oder den mehreren nichtlinearen Maschinenlernmodellen; und
Ausgeben (512) eines Pflanzennährstoffberichts, der die verfeinerten Blattstielnährstoffwerte angibt,
wobei das eine oder die mehreren nichtlinearen Maschinenlernmodelle dazu trainiert sind, Zusammenhänge in den Nährstoffaufnahmeeigenschaften von unterschiedlichen Pflanzennährstoffen zu modellieren.

10. System nach Anspruch 9, wobei der Prozessor ferner dazu konfiguriert ist, die Spektraldaten, die von einem Blatt (18) einer Pflanze (10) unter Verwendung eines Spektrophotometers (104) genommen werden, zu empfangen.

11. System nach einem der Ansprüche 9-10, wobei jedes von dem einen oder den mehreren nichtlinearen Maschinenlernmodellen ein multivariates Regressionsmodell ist.

12. System nach einem der Ansprüche 9-11, wobei der Prozessor ferner dazu konfiguriert ist, eine Düngemittelbehandlung auf Pflanzen zumindest teilweise auf Basis der verfeinerten Blattstielnährstoffwerte anzuwenden (514).

## Revendications

1. Procédé (500) de détermination de valeurs nutritives de pétiole affinées sur la base de données spectrales de feuilles provenant de feuilles (18) de plantes, le procédé comprenant :
(a) le stockage (506) des données spectrales de feuilles dans une mémoire (120) ;
(b) la transformation (508), à l'aide d'un processeur (116), des données spectrales de feuilles en valeurs nutritives de pétiole préliminaires en entrant les données spectrales de feuilles dans un ou plusieurs modèles d'apprentissage automatique linéaires entraînés pour fournir des sorties de valeurs nutritives de pétiole sur la base des entrées de données spectrales de feuilles, le ou les modèles d'apprentissage automatique linéaires émettant en sortie les valeurs nutritives de pétiole préliminaires ;
**caractérisé par** :
(c) l'affinage (510), à l'aide du processeur, des valeurs nutritives de pétiole préliminaires en entrant les valeurs nutritives de pétiole préliminaires dans un ou plusieurs modèles d'apprentissage automatique non linéaires entraînés pour fournir des sorties de valeurs nutritives de pétiole affinées sur la base des entrées de valeurs nutritives de pétiole préliminaires, le ou les modèles d'apprentissage automatique non linéaires émettant en sortie des valeurs nutritives de pétiole affinées ; et
(d) l'émission en sortie (512) d'un rapport sur les éléments nutritifs de plantes indiquant les valeurs nutritives de pétiole affinées,
ledit ou lesdits modèles d'apprentissage automatique non linéaires étant entraînés pour modéliser des interrelations dans les caractéristiques d'absorption d'éléments nutritifs de différents éléments nutritifs de plante.

2. Procédé (500) de la revendication 1, comprenant en outre, avant ledit stockage, la prise (504) de mesures spectrales d'une feuille (18) d'une plante (10) à l'aide d'un spectrophotomètre (104) pour générer des données spectrales de feuilles.

3. Procédé (500) d'une quelconque revendication précédente, chacun du ou des modèles d'apprentissage automatique non linéaires étant un modèle de régression multivarié.

4. Procédé (500) d'une quelconque revendication précédente, comprenant en outre l'application (514) d'un traitement d'engrais aux plantes sur la base au moins en partie des valeurs nutritives de pétiole affinées.

5. Support non transitoire lisible par ordinateur comprenant des instructions exécutables par un processeur (116), lesdites instructions, lorsqu'elles sont exécutées, configurant le processeur pour :
stocker (506) des données spectrales de feuilles provenant de feuilles (18) de plantes dans une mémoire (120) ;
transformer (508), à l'aide du processeur, les données spectrales de feuilles en valeurs nutritives de pétiole préliminaires en entrant les données spectrales de feuilles dans un ou plusieurs modèles d'apprentissage automatique linéaires entraînés pour fournir des sorties de valeurs nutritives de pétiole sur la base des entrées de données spectrales de feuilles ;
recevoir une sortie comprenant les valeurs nutritives de pétiole préliminaires en provenance du ou des modèles d'apprentissage automatique linéaires ;
affiner (510), à l'aide du processeur, les valeurs nutritives de pétiole préliminaires en entrant les valeurs nutritives de pétiole préliminaires dans un ou plusieurs modèles d'apprentissage automatique non linéaires entraînés pour fournir des sorties de valeurs nutritives de pétiole affinées sur la base des entrées de valeurs nutritives de pétiole préliminaires ;
recevoir une sortie comprenant les valeurs nutritives de pétiole affinées en provenance du ou des modèles d'apprentissage automatique non linéaires ; et
émettre en sortie (512) un rapport sur les éléments nutritifs de plantes indiquant les valeurs nutritives de pétiole affinées,
ledit ou lesdits modèles d'apprentissage automatique non linéaires étant entraînés pour modéliser des interrelations dans les caractéristiques d'absorption d'éléments nutritifs de différents éléments nutritifs de plante.

6. Support non transitoire lisible par ordinateur de la revendication 5, lesdites instructions, lorsqu'elles sont exécutées, configurant le processeur pour recevoir les données spectrales prises d'une feuille (18) d'une plante (10) à l'aide d'un spectrophotomètre (104).

7. Support non transitoire lisible par ordinateur de l'une quelconque des revendications 5-6, chacun du ou des modèles d'apprentissage automatique non linéaires étant un modèle de régression multivarié.

8. Support non transitoire lisible par ordinateur de l'une quelconque des revendications 5-7, lesdites instructions, lorsqu'elles sont exécutées, configurant le processeur pour appliquer (514) un traitement d'engrais aux plantes sur la base au moins en partie des valeurs nutritives de pétiole affinées.

9. Système destiné à la détermination de valeurs nutritives de pétiole affinées sur la base de données spectrales de feuilles provenant de feuilles (18) de plantes, le système comprenant :
une mémoire (120) configurée pour stocker des données spectrales de feuilles provenant de feuilles (18) de plantes ; et
un processeur (116) configuré pour :
transformer (508) les données spectrales de feuilles en valeurs nutritives de pétiole préliminaires en entrant les données spectrales de feuilles dans un ou plusieurs modèles d'apprentissage automatique linéaires entraînés pour fournir des sorties de valeurs nutritives de pétiole sur la base des entrées de données spectrales de feuilles ;
recevoir une sortie comprenant les valeurs nutritives de pétiole préliminaires en provenance du ou des modèles d'apprentissage automatique linéaires ;
**caractérisé en ce que** le processeur est en outre configuré pour :
affiner (510) les valeurs nutritives de pétiole préliminaires en entrant les valeurs nutritives de pétiole préliminaires dans un ou plusieurs modèles d'apprentissage automatique non linéaires entraînés pour fournir des sorties de valeurs nutritives de pétiole affinées sur la base des entrées de valeurs nutritives de pétiole préliminaires ;
recevoir une sortie comprenant les valeurs nutritives de pétiole affinées en provenance du ou des modèles d'apprentissage automatique non linéaires ; et
émettre en sortie (512) un rapport sur les éléments nutritifs de plantes indiquant les valeurs nutritives de pétiole affinées,
ledit ou lesdits modèles d'apprentissage automatique non linéaires étant entraînés pour modéliser des interrelations dans les caractéristiques d'absorption d'éléments nutritifs de différents éléments nutritifs de plante.

10. Système de la revendication 9, ledit processeur étant en outre configuré pour recevoir les données spectrales prises d'une feuille (18) d'une plante (10) à l'aide d'un spectrophotomètre (104).

11. Système de l'une quelconque des revendications 9-10, chacun du ou des modèles d'apprentissage automatique non linéaires étant un modèle de régression multivarié.

12. Système de l'une quelconque des revendications 9-11, ledit processeur étant en outre configuré pour appliquer (514) un traitement d'engrais aux plantes sur la base au moins en partie des valeurs nutritives de pétiole affinées.
